(19) Europäisches Patentamt
European Patent Office
Office européen des brevets

(11) EP 0 671 386 B1

(12) **FASCICULE DE BREVET EUROPEEN**

(45) Date de publication et mention de la délivrance du brevet:
**04.11.1998 Bulletin 1998/45**

(51) Int. Cl.$^6$: **C07C 311/03**, C08G 65/32, H01M 10/40

(21) Numéro de dépôt: **95400458.6**

(22) Date de dépôt: **03.03.1995**

(54) **Monomères dérivés de sultones perhalogénées et polymères obtenus à partir de ces monomères**

Von perhalogenierten Sultonen abgeleiteten Monomere, sowie aus diesen Monomeren hergestellte Polymere

Monomers derived from perhalogeneted sultones and polymers derived therefrom

(84) Etats contractants désignés:
**DE FR GB IT**

(30) Priorité: **09.03.1994 US 207608**

(43) Date de publication de la demande:
**13.09.1995 Bulletin 1995/37**

(73) Titulaires:
- **CENTRE NATIONAL DE LA RECHERCHE SCIENTIFIQUE (CNRS)**
  **75016 Paris (FR)**
- **HYDRO-QUEBEC**
  **Montréal Québec H2Z 1A4 (CA)**

(72) Inventeurs:
- **Armand, Michel**
  **F-38410 Saint-Martin-D'Uriage (FR)**
- **Sanchez, Jean-Yves**
  **F-38330 Saint-Ismier (FR)**
- **Sylla, Salime**
  **Bamako (ML)**

(74) Mandataire: **Sueur, Yvette et al**
**Cabinet SUEUR & L'HELGOUALCH,**
**78, Rue Carnot**
**95240 Cormeilles-en-Parisis (FR)**

(56) Documents cités:
**FR-A- 2 687 671** **US-A- 4 329 478**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 671 386 B1

## Description

La présente invention concerne des monomères dérivés de sultones perhalogénées, leur procédé de préparation, les polymères obtenus à partir des dits monomères et leur utilisation pour l'élaboration de matériaux à conduction ionique.

On connaît des composés $ROCF_2CF(CF_3)SO_2F$ dans lesquels R est un groupement alkyle éventuellement halogéné comportant une double liaison vinylique, une liaison acétylénique, un groupe époxy ou des groupements $(CH_3-CH_2-O)_2P(O)-CH_2-CH_2-$ ou $CH_3CH_2-O-P-F(O)-CH_2-CH_2$, ainsi que leur procédé de préparation à partir d'une sultone fluorée cyclique (Chen et al, J. of Fluorine Chemistry, 48 (1990) 107-122) ; Chen et al, J. of Fluorine Chemistry, vol.46, 1990, p.21-38 ; Chen et al, J. of Fluorine Chemistry, vol.46, 1990, p.39-56). Des composés dans lesquels un groupe $-SO_3M$, M étant H, Li, Na, $NH_4$, K, est fixé sur un radical $R^1R^2-N-C(O)-CH-(CH_2)_n-COOM$, n étant 0 ou 1, $R^1$ et $R^2$ étant des groupes alkyles, sont décrits dans US-A-4490308 (D.W. Fong et al). EP-A-0124378 (Du Pont De Nemours) décrit des monomères $CH_2 = CH-CF_2-CF_2-OCF_2-CF_2-SO_2F$, des copolymères de ces monomères avec de l'éthylène, l'utilisation de ces copolymères comme matériaux isolants électriques. Dans Chem. Abs., vol. 114, 1991, n°23718b, Huaxue, sont décrits des composés résultant de la réaction d'un fluorure de difluoroiodo-méthane sulfonyle avec une oléfine en présence de cuivre, par exemple $nBu-CH = CH-CF_2-SO_2F$, $Me-C(CH_2)-C(CH_3)_2-CF_2\ SO_2F$ ou $CH_2 = CH-(CH_2)_2-CH = CH-CF_2\ SO_2F$.

Par le brevet européen N°13199 (Armand et Duclot), on connaît les électrolytes polymères obtenus par dissolution d'un sel $M^+X^-$ dans un polymère solvatant comportant des hétéroatomes, $M^+$ représentant $H^+$, un cation métallique, un cation organique du type ammonium, amidinium ou guanidinium; X représentant un anion à charge électronique délocalisée, par exemple $Br^-$, $ClO_4^-$, $R_FSO_3^-$ ou $(R_FSO_2)_2N^-$ ; $R_F$ représentant un groupement perfluoroalkyle ou perfluoroaryle. Ces électrolytes polymères ont de nombreuses applications, en particulier dans le domaine des générateurs électrochimiques, des systèmes de modulation de la lumière (M. Armand et al, EP-87401555), des capteurs, par exemple pour des membranes sélectives ou de référence (A. Hammou et al, FR-86.09602). De nombreux travaux ont été effectués, en particulier pour l'amélioration des propriétés de conduction de ces matériaux. Ils ont abouti par exemple à la formation de copolymères à base d'oxyde d'éthylène (M. Armand et al, FR-83.09886) ou de réseaux réticulés par des ponts uréthanes (H. Chéradame et al, FR-8007135, US-4,357,401). Ces matériaux ont cependant pour caractéristique commune de présenter une mobilité de l'anion $X^-$ qui est, dans la plupart des cas, supérieure à celle du cation $M^+$. Cette propriété est un inconvénient, en particulier pour les systèmes électrochimiques pour lesquels la réaction d'électrode fait intervenir le cation $M^+$. Le passage du courant entraîne l'établissement d'un gradient de concentration du sel dissous qui augmente la résistivité de l'électrolyte et peut amener la formation à proximité des interfaces, de composés stoechiométriques polymère-sel présentant une conductivié ionique insuffisante.

Plusieurs tentatives ont été faites pour synthétiser des polymères contenant des groupements anioniques attachés à la chaîne macromoléculaire. Les ionophores contenant des groupements alkylsulfonates $RSO_3^-$, des groupements carboxylates ou des groupements perfluorocarboxylates $-R_FCO_2^-$ [D.J. Bannister et al, Polymer **25**, 1291 (1984) ; E. Tsushida et al, Macromolécules **21**, 96 (1988) ] sont cependant très peu dissociés dans les polymères solvatants de type polyéther comme le polyoxyde d'éthylène. Les conductivités ioniques obtenues avec de tels matériaux sont ainsi très faibles et ne permettent pas d'envisager d'applications pratiques.

Par ailleurs, des groupements perfluorosulfoniques $-R_FSO_3^-$ constituent les groupements ionophores des membranes échangeuses d'ions de type NAFION®. De telles membranes, ainsi que les monomères à partir desquels elles sont obtenues, sont décrits par exemple dans DE-A-3047439 (Asahi Kasei Kogyo KK), DE-A-2461675 (Du Pont De Nemours), US-A-3718627 (W.G. Grot) ou dans US-A-3714245 (R. Beckerbauer). La partie réticulable des monomères ainsi que le squelette des polymères constituant les membranes sont perfluorés. Ces polymères ne présentent de conductivité ionique qu'en présence de solvants polaires tels que l'eau, les alcools, le carbonate de propylène ; la trame perfluorée de ce type de polymère n'a en effet aucun caractère solvatant ni dissociant, contrairement aux polyéthers.

Des polymères $-(CX_2CXR)_n-$ ou $-(CXRCXR)_n-$ dans lequels X est H ou F et R représente $-(CX_2)_m-SO_3H$, ainsi que leur utilisation comme électrolyte dans des piles à combustible sont décrits dans Chem. Abstr., vol. 117, 1992, n°27945h Matsushita.

WO-A-9202571 (SRI International) décrit des polymères à conduction ionique portant des anions greffés $-X-Y^-M^+$, X pouvant être $CF_2$ ou $CFR_F$, Y pouvant être $SO_3$ et $M^+$ étant un cation. Le squelette des polymères est dérivé d'un polymère linéaire ayant des hydrogènes mobiles. Il peut s'agir par exemple d'un squelette du type polyéther, polyester, poly(éthylène)imine, polyphosphazène, siloxane. Les polymères peuvent être obtenus soit en greffant les groupes appropriés sur n'importe quelle chaîne polymérique ayant des hydrogènes mobiles, soit en polymérisant des monomères à cycle oxazoline portant un groupe $-SO_2F$.

FR-A-2 687 671 décrit des monomères $A-CFX-SO_2Z$ dans lesquels A représente l'un des groupes $R^1R^2N-CO-$, $R^3-O-CF_2-$ ou $R^3-$ ; Z représente F, Cl, $-OSi(CH_3)_3$ ou un groupement ionique ; x représente F, Cl, H ou $R_F$ ; les radicaux $R^1$, $R^2$ et $R_3$ sont choisis parmi les radicaux organiques non perfluorés polymérisables ; RF est choisi parmi les radicaux perfluoroalkyle et les radicaux perfluoroaryle. Ces monomères peuvent être utilisés pour l'obtention de polymères

portant des groupes ioniques pendants $-SO_2Z$ qui présentent des propriétés de conduction ionique.

La présente invention a pour but de fournir des monomères permettant d'obtenir des polymères particulièrement intéressants pour l'élaboration de matériaux à conduction cationique.

C'est ainsi que la présente invention a pour objet des monomères perhalogénés.

Elle a également pour objet un procédé de préparation de ces monomères.

Elle a en outre pour objet les polymères obtenus à partir des dits monomères.

Enfin, elle a pour objet des matériaux à conduction ionique contenant les dits polymères. Les monomères de la présente invention sont des composés répondant à la formule générale (1) :

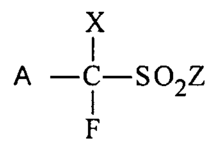

dans laquelle:

- A représente

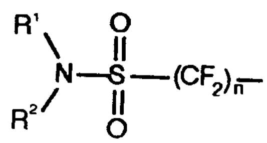

- Z représente F, Cl, $-OSi(CH_3)_3$ ou un groupement ionique ;
- X représente F, Cl, H ou $R_F$ ;
- l'un au moins des radicaux $R^1$ et $R^2$ est choisi parmi les radicaux organiques non perfluorés polymérisables, $R^1$ ou $R^2$ étant un radical organique non perfluroré lorsqu'il est non polymérisable ;
- $R_F$ est choisi parmi les radicaux perfluoroalkyle et les radicaux perfluoroaryle ;
- n = 1, 2 ou 3 ; les valeurs préférées étant 1 ou 2 lorsque X = F.

Les groupements ioniques Z particulièrement préférés sont choisis parmi les groupements $[-O^-, 1/mM^{m+}]$, $[-N(SO_2\text{-}Q)^-, 1/mM^{m+}]$, $[-CH(SO_2\text{-}Q)^-, 1/mM^{m+})]$ et $[-C(SO_2\text{-}Q)_2^-, 1/mM^{m+}]$, Q représentant $-R_F$ ou -CFX-A ; $M^{m+}$ représentant un ion d'un métal ayant la valence m, choisi parmi les métaux alcalins, les métaux alcalino-terreux, les métaux de transition et les terres rares, ou un ion hydronium, ou un ion ammonium répondant à la formule $NH_{(4-j)}R_j^+$, ou un ion amidinium répondant à la formule $RC(NH_{2-j}R_j)_2^+$, ou un ion guanidinium répondant à la formule $C(NH_{2-j}R_j)_3^+$, avec j = 0, 1 ou 2, R étant choisi parmi l'hydrogène, un groupement alkyle, oxaalkyle ou aryle.

Parmi les différents cations $M^{m+}$, ceux qui correspondent à des réactions d'électrodes, par exemple dans les systèmes de stockage d'énergie, de modulation du rayonnement électromagnétique, les électrodes de référence, sont particulièrement intéressants. A cet égard, les ions $Li^+$, $Na^+$, $K^+$, $Cs^+$, $NH_4^+$, $Ca^{++}$, $Cu^{++}$, $Zn^{++}$, $Mg^{++}$ sont particulièrement préférés.

Parmi les groupements $R_F$ du type perfluoroalkyle, on préfère les radicaux perfluoroalkyle ayant de 1 à 8 atomes de carbone, et plus particulièrement les radicaux perfluoroalkyle ayant de 1 à 4 atomes de carbone. On peut citer les radicaux $CF_3$-, $C_2F_5$-, n-$C_3F_7$-, n-$C_4F_9$-.

Parmi les groupements $R_F$ du type perfluoroaryle, on préfère les radicaux perfluoroaryle ayant de 6 à 8 atomes de carbone, par exemple le radical perfluorophényle.

Les groupements organiques non perfluorés polymérisables $R^1$ et $R^2$ permettent des réactions de polymérisation par voie radicalaire, anionique, cationique ou stéréospécifique, ou par polycondensation. Ils peuvent être choisis parmi ceux qui comportent des doubles liaisons, par exemple des doubles liaisons du type vinyle, allyle, vinylbenzyle ou acryloyle. Ils peuvent également être choisis parmi ceux qui comportent des fonctions oxirane, oxétane, azétidine ou aziridine. En outre, ils peuvent être choisis parmi ceux qui comportent des fonctions alcool, thiol, amine, isocyanate ou trialcoxysilane. Ils peuvent également être choisis parmi ceux qui comportent des fonctions permettant une électropolymérisation.

Le substituant R des groupements ammonium, amidinium ou guanidinium est choisi de préférence parmi H, les groupements alkyles ayant de 1 à 20 atomes de carbone, les groupement oxalkyle ayant de 1 à 20 atomes de carbone ou les groupements aryle ayant de 6 à 30 atomes de carbone. On préfèrera tout particulièrement les groupements méthyle, éthyle, propyle, lauryle et méthoxyéthyle.

Parmi les monomères de la présente invention, on préfère tout particulièrement ceux qui répondent à la formule générale (1), dans laquelle Z représente un groupement ionique. A titre d'exemple, on peut citer les familles suivantes :

$1/mM^{m+}[A\text{-}CFX\text{-}SO_3]^-$,
$1/mM^{m+}[A\text{-}CFX\text{-}SO_2\text{-}N\text{-}SO_2\text{-}R_F]^-$,
$1/mM^{m+}[A\text{-}CFX\text{-}SO_2\text{-}CH(SO_2\text{-}R_F)]^-$,
$1/mM^{m+}[A\text{-}CFX\text{-}SO_2\text{-}C(SO_2\text{-}R_F)_2]^-$,
$1/mM^{m+}[A^1\text{-}CFX^1\text{-}SO_2\text{-}N\text{-}SO_2\text{-}CFX^2\text{-}A^2]^-$,
$1/mM^{m+}[A^1\text{-}CFX^1\text{-}SO_2\text{-}CH(SO_2\text{-}CFX^2\text{-}A^2)]^-$,
$1/mM^{m+}[A^1\text{-}CFX^1\text{-}SO_2\text{-}C(SO_2\text{-}CFX^2\text{-}A^2)_2]^-$,

$A^1$ et $A^2$, identiques ou différents, étant choisis parmi les groupements A définis précédemment ; $X^1$ et $X^2$, identiques ou différents, étant choisis parmi les groupements X cités précédemment.
Les composés monomères suivants, dans lesquels n est 2 ou 3, sont particulièrement intéressants :

$Li\ [(H_2C{=}CH\text{-}CH_2)_2N\text{-}SO_2\text{-}(CF_2)_nSO_3]$
$Li\ [(H_2C{=}CH\text{-}CH_2)_2N\text{-}SO_2\text{-}(CF_2)_nSO_2]_2N$
$Li\ [(H_2C{=}CH\text{-}CH_2)_2N\text{-}SO_2\text{-}(CF_2)_nSO_2]_3C$
$Li\ [(H_2C{=}CH\text{-}CH_2)_2N\text{-}SO_2\text{-}(CF_2)_nSO_2]_2CH$
$Li\ [(H_2C{=}CH\text{-}CH_2)_2N\text{-}SO_2\text{-}(CF_2)_nSO_2\text{-}C(SO_2CF_3)_2]$

Un composé de la présente invention dans lequel X est F et Z est $[-O^-, 1/mM^+]$, désigné ci-après par composé (3), peut être préparé par ouverture du cycle d'un anhydride cyclique (2) avec une amine $R^1R^2NH$, selon le schéma réactionnel :

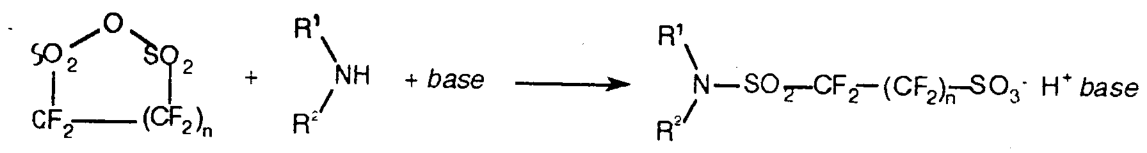


la base étant soit une base azotée tertiaire, sous l'amine précitée $R^1R^2NH$, puis remplacement du groupe ($H^+$base) par $1/mM^+$ par une technique classique d'échange d'ion.

Un halogénure de sulfonyle $R^1R^2N\text{-}SO_2\text{-}(CF_2)_nSO_2Z$ dans lequel Z représente un halogène, qui sera désigné ci-après par composé (4), peut être préparé par un procédé consistant à traiter l'un des composés $R^1R^2N\text{-}SO_2\text{-}(CF_2)_nSO_2Z$ dans lequel Z représente $[-O^-, 1/mM^+]$ ou $[-O^-, (H^+base)]$ par un agent d'halogénation. L'agent d'halogénation peut être choisi parmi le tétrafluorure de soufre, le chlorure de thionyle, le chlorure d'oxalyle, le trifluorure de diéthylaminosoufre, ou d'autres agents d'halogénation connus.

Un dérivé ayant l'une des formules suivantes :

$1/mM^{m+}\ [R^1R^2N\text{-}SO_2\text{-}(CF_2)_{n+1}SO_2\text{-}N\text{-}SO_2\text{-}Q]^-$ $1/mM^{m+}\ [R^1R^2N\text{-}SO_2\text{-}(CF_2)_{n+1}SO_2\text{-}CH\text{-}SO_2\text{-}Q]^-$ $1/mM^{m+}\ [R^1R^2N\text{-}SO_2\text{-}(CF_2)_{n+1}SO_2\text{-}C(SO_2\text{-}Q)_2]^-$

peut être préparé à partir de l'halogénure de sulfonyle correspondant $R^1R^2N\text{-}SO_2\text{-}(CF_2)_nSO_2Z$ (4) par l'un des procédés suivants.

Dans la suite du texte, A représente le groupement $R^1R^2N\text{-}SO_2\text{-}(CF_2)_n$.

Un composé (5c) dans lequel Z est $-OSi(CH_3)_3$ est obtenu par réaction d'un composé $A\text{-}CFX\text{-}SO_2F$ (4') avec un agent de silylation, en particulier l'hexaméthyldisiloxane selon la réaction :

$$A\text{-}CFX\text{-}SO_2F + (CH_3)_3SiOSi(CH_3)_3 \rightarrow A\text{-}CFX\text{-}SO_3Si(CH_3)_3 + FSi(CH_3)_3$$

Un composé (5d) pour lequel Z = [-N($SO_2$Q)$^-$, 1/mM$^{m+}$] est obtenu par réaction du composé (4') correspondant avec 1/mM$^{m+}$[HN$SO_2$Q]$^-$ en présence d'une base nucléophile.

Un composé (5e) pour lequel Z = [-CH($SO_2$Q)$^-$, 1/mM$^{m+}$] est obtenu par réaction du composé (4') correspondant avec 1/mM$^{m+}$[$H_2$C($SO_2$Q)]$^-$ en présence d'une base nucléophile.

Un composé (5f) pour lequel Z = [-C($SO_2$Q)$_2^-$, 1/mM$^{m+}$] est obtenu par réaction du composé (4') correspondant avec 1/mM$^{m+}$[HC($SO_2$Q)$_2$]$^-$ en présence d'une base nucléophile. Dans les composés (5d) à (5f) ci-dessus, Q représente A-CFX-ou $R_F$-.

La base nucléophile est choisie de préférence parmi les trialkylamines [par exemple la triéthylamine, la diisopropyléthylamine, la quinuclidine, le 1,4 diazabicyclo[2,2,2]octane (TED)], les pyridines (par exemple la pyridine, les alkylpyridines, les dialkylaminopyridines), les imidazoles (par exemple les N-alkylimidazoles, l'imidazo [1,2-a]pyridine), les amidines, par exemple le 1,5-diazabicyclo[4,3,0]non-5-ène (DBN) et le 1,8 diazabicyclo [5,4,0] undec-7-ène (DBU)], les guanidines [par exemple la tétra méthylguanidine et la 1,3,4,7,8 hexahydro-1-méthyl-2H-pyrimido[1,2-a]-pyrimidine (HPP)].

Lorsqu'un composé monomère de la présente invention est un composé symétrique tel que 1/mM$^{m+}$[(A-CFX-$SO_2$)$_2$N]$^-$, 1/mM$^{m+}$[(A-CFX-$SO_2$)$_2$CH]$^-$ ou 1/mM$^{m+}$[(A-CFX-$SO_2$)$_3$C]$^-$, il peut être préparé par action directe du fluorure d'acide sulfonylacétique A-CFX-$SO_2$F sur le nitrure ionique $Li_3N$ ou le carbure ionique $C_3Al_4$ dans un solvant aprotique polaire.

Bien entendu, les monomères de la présente invention dans lesquels Z représente un groupement ionique obtenus par l'un des procédés décrits ci-dessus peuvent être modifiés par les procédés classiques d'échange de cations, par exemple à l'aide de résines échangeuses d'ions, en vue de remplacer le cation M par un autre cation.

Lorsque les groupes polymérisables existant dans les groupements $R^1$ et $R^2$ présentent des fonctions dont la réactivité pourrait interférer avec les réactions de préparation des monomères telles que décrites ci-dessus, les dites fonctions peuvent être protégées par des réactifs qui leurs sont liés d'une manière réversible.

Les composés monomères de la présente invention peuvent être polymérisés grâce aux fonctions polymérisables présentes dans les groupements $R^1$ et $R^2$. Les polymères obtenus selon la présente invention portent des fonctions -$SO_2$Z.

Selon une première variante, on obtient un polymère linéaire dont la trame est constituée par les groupements $R^1$ et $R^2$ polymérisés et porte des greffons présentant des terminaisons -$SO_2$Z. Ce polymère est obtenu par homopolymérisation d'un composé monomère de la présente invention. Les composés monomères de la présente invention peuvent être homopolymérisés lorsque le groupement A comporte au moins deux fonctions réactives complémentaires. A titre d'exemple, on peut citer les groupements A comportant un groupe diallyle amino, vinylphénylamino ou acrylamidoéthyl amino.

Selon une deuxième variante, les polymères de la présente invention sont obtenus sous forme de copolymères à partir d'au moins un monomère de la présente invention et d'un ou plusieurs comonomères. Au moins l'un des comonomères est choisi de telle sorte qu'il comporte une fonction capable de réagir avec le groupement polymérisable du monomère selon l'invention. Les comonomères peuvent en outre être choisis en fonction des propriétés particulières qu'ils conféreront au copolymère. A cet égard, on peut également introduire dans le copolymère, des monomères additionnels ne réagissant pas avec le monomère de l'invention, mais intéressants par leurs propriétés.

Les copolymères peuvent être obtenus par deux voies différentes. Suivant une première voie, on obtient un polymère contenant des unités monomères de la présente invention en coréticulant les composés monomères de la présente invention avec un homopolymère ou un copolymère préexistant comportant des fonctions capables de réagir avec la fonction portée par l'un au moins des groupements $R^1$ et $R^2$. Les réseaux macromoléculaires ainsi obtenus sont constitués par une trame macromoléculaire constituée par l'homopolymère ou le copolymère préexistant sur laquelle sont greffées les unités monomères selon l'invention, comportant un groupe -$SO_2$Z. Comme exemple de polymères préexistants, on peut citer les polymères obtenus par polymérisation d'allylglycidyléther, éventuellement en présence d'autres monomères tels que l'oxyde d'éthylène ou le méthyl glycidyléther, les copolymères butadiène-acrylonitrile, un copolymère de diisocyanate de $\alpha,\omega$ diaminooligooxyéthylène et de poly(oxyéthylène-triol). Suivant une deuxième voie, les composés monomères de la présente invention peuvent être incorporés dans des polymères par copolymérisation directe avec un ou plusieurs co-monomères dont l'un au moins porte des fonctions capables de réagir avec la fonction portée par l'un au moins des groupements $R^1$ et $R^2$. Parmi ces monomères capables de polymériser avec un monomère de l'invention, on peut citer l'allylglycidyl éther, les époxyalcènes, les acrylates de glycidyle; parmi les monomères non réactifs on peut citer l'oxyde d'éthylène, l'oxyde de propylène, le méthylglycidyl éther.

Dans tous les cas, lorsque le monomère selon l'invention choisi est du type symétrique, c'est-à-dire lorsque le substituant Z comporte un groupement A-CFX-, qui peut être identique ou différent du groupement A-CFX- provenant du fluorure de l'acide acétylsulfonique de départ, les copolymères obtenus sont réticulés.

Lorsque les polymères de la présente invention sont obtenus à partir de monomères selon l'invention comportant un groupement Z ionique, ils peuvent être traités de manière classique, par exemple à l'aide de résines échangeuses de cations, pour remplacer un cation $M^{m+}$ par un cation différent.

Les polymères et les réseaux macromoléculaires de la présente invention sont utiles pour l'élaboration de matériaux à conduction ionique.

Les polymères ou les réseaux macromoléculaires incorporant des monomères selon la présente invention sont particulièrement intéressants lorsque Z représente un groupement ionique choisi parmi [-O$^-$, 1/mM$^{m+}$], [-N(SO$_2$Q)$^-$, 1/mM$^{m+}$], [-CH(SO$_2$Q)$^-$, 1/mM$^{m+}$] et [-C(SO$_2$Q)$_2$$^-$ , 1mM$^{m+}$], Q représentant $R_F$ ou [$R^1R^2$N-SO$_2$-(CF$_2$)$_n$CF(X)-]. Dans ce cas, ils portent des anions greffés sur la chaîne macromoléculaire et constituent en eux-même des matériaux à conduction ionique sans adjonction supplémentaire de sels. La présence de sels facilement dissociables peut toutefois être utile.

Lorsque les polymères et les réseaux macromoléculaires de la présente invention portent un substituant Z qui n'est pas un groupement ionique, il est nécessaire d'y ajouter un sel facilement dissociable lors de l'élaboration d'un matériau à conduction ionique.

Les matériaux à conduction ionique de l'invention, et plus spécialement ceux dans lesquels les anions sont fixés sur la chaîne macomoléculaire, sont particulièrement utiles dans les systèmes électrochimiques pour lesquels la réaction d'électrode fait intervenir les cations, par exemple dans les cellules électrochimiques comportant une anode contenant du lithium. Parmi ces systèmes électrochimiques, on peut citer les générateurs électrochimiques au lithium, les systèmes électrochromes, ou encore les capteurs comportant des membranes sélectives ou des membranes de référence.

La présente invention est décrite plus en détail dans les exemples suivants, donnés à titre illustratif, mais non limitatif.

EXEMPLE 1

L'anhydride cyclique de l'acide perfluoro propane-1,3-disulfonique est préparé par fluoration de F-SO$_2$-(CH$_2$)$_3$-SO$_2$F, hydrolyse du produit obtenu et déshydratation, selon le mode opératoire décrit par exemple par F. Behr, T. Kestner, Abstract n° 56, 10th International Symposium on Fluorine Chemistry, University of British Columbia, Vancouver, Colombie Britannique, 1-6 août 1982. La préparation du composé F-SO$_2$-(CH$_2$)$_3$-SO$_2$F est décrite par exemple par E. Hollitzer, P. Sartory, J. of Fluorine Chemistry, n° 35, p. 329-341.

On dilue 30 g de l'anhydride cyclique de l'acide perfluoro propane-1,3- disulfonique dans 200 ml de dichlorométhane et on refroidit le mélange à -10 °C. On ajoute ensuite goutte à goutte 20 g de diallylamine en maintenant une bonne agitation mécanique. Le mélange réactionnel est ensuite ramené à température ordinaire, en maintenant l'agitation, et le dichlorométhane est éliminé dans un évaporateur rotatif. On ajoute ensuite au produit résiduel 4,5 g d'hydroxyde de lithium LiOH.H$_2$O dans 30 ml d'eau. Le mélange est à nouveau soumis à une évaporation et le résidu est extrait par 100 ml de THF distillé.

Le schéma réactionnel de formation du sel est le suivant :

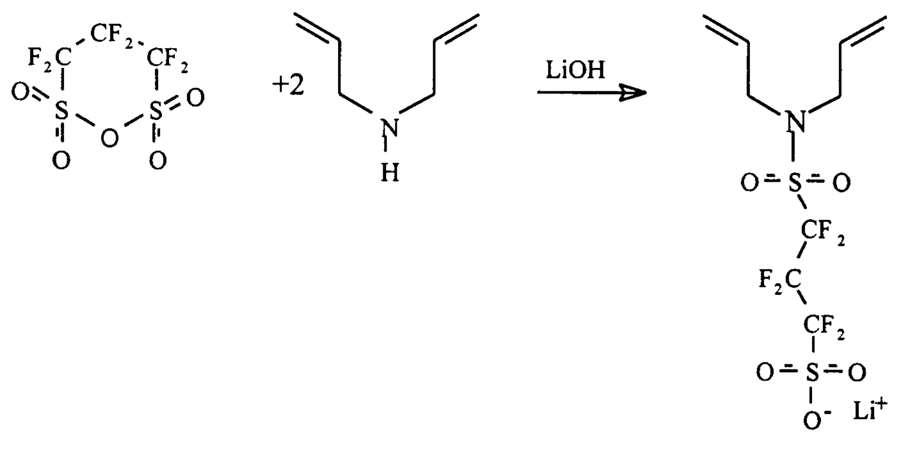


Le sel obtenu est purifié par recristallisation dans un mélange isopropanol-dichloroéthane.

EXEMPLE 2

On dissout 1,5 g du sel obtenu dans l'exemple 1 et 11,5 g d'un copolymère d'oxyde d'éthylène et d'allylglycidyléther dans 70 ml d'acétonitrile, puis on ajoute 150 mg de peroxyde de benzoyle.

Un film de complexe polymère-sel d'une épaisseur de 4 µm est obtenu par coulage de la solution dans un anneau de verre posé sur une plaque de poly(tétrafluoroéthylène), suivi d'une évaporation du solvant. Le film est ensuite chauffé sous atmosphère d'argon à 80 °C pendant 2 heures. Le film réticulé obtenu est un électrolyte polymère à conduction cationique. La conduction, mesurée à 60 °C est supérieure à $10^{-5}$ $S.cm^{-1}$.

## Revendications

**1.** Composé répondant à la formule générale (1) suivante

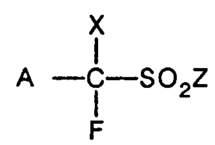

dans laquelle :

- A représente

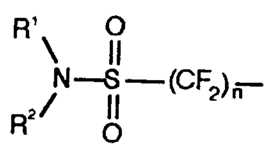

- Z représente F, Cl, $-OSi(CH_3)_3$ ou un groupement ionique ;
- X représente F, Cl, H ou $R_F$ ;
- l'un au moins des radicaux $R^1$ et $R^2$ est choisi parmi les radicaux organiques non perfluorés polymérisables, $R^1$ ou $R^2$ étant un radical organique non perfluroré lorsqu'il est non polymérisable ;
- $R_F$ est choisi parmi les radicaux perfluoroalkyle et les radicaux perfluoroaryle ;
- n = 1, 2 ou 3 ; les valeurs préférées étant 1 ou 2 lorsque X = F.

**2.** Composé selon la revendication 1, caractérisé en ce que Z représente un groupement ionique choisi parmi $[-O^-, 1/mM^{m+}]$, $[-N(SO_2-Q)^-, 1/mM^{m+}]$, $[-CH(SO_2-Q)^-, 1/mM^{m+})]^-$ et $[-C(SO_2-Q)_2^-, 1/mM^{m+}]^-$, Q représentant $-R_F$ ou -CFX-A ; $M^{m+}$ représentant un ion d'un métal ayant la valence m, choisi parmi les métaux alcalins, les métaux alcalino-terreux, les métaux de transition et les terres rares, ou un ion hydronium ou un ion ammonium répondant à la formule $NH_{(4-j)}R_j^+$, ou un ion amidinium répondant à la formule $RC(NH_{2-j}R_j)_2^+$, ou un ion guanidinium répondant à la formule $C(NH_{2-j}R_j)_3^+$, avec j = 0, 1 ou 2, R étant choisi parmi l'hydrogène, un groupement alkyle, oxaalkyle ou aryle.

**3.** Composé selon l'une quelconque des revendications 1 ou 2, caractérisé en ce que $R_F$ est choisi parmi les groupements perfluoroalkyle ayant de 1 à 8 atomes de carbone, et les groupements perfluoroaryle ayant de 6 à 8 atomes de carbone.

**4.** Composé selon la revendication 1, caractérisé en ce que les groupements $R^1$ ou $R^2$ comportent des doubles liaisons du type vinyle, allyle, vinylbenzyle ou acryloyle, ou une fonction oxirane, une fonction oxétane, une fonction azétidine, une fonction aziridine, une fonction alcool, une fonction thiol, une fonction amine, une fonction isocyanate ou une fonction trialcoxysilane.

**5.** Composé selon la revendication 1, caractérisé en ce qu'il répond à l'une des formules suivantes :

$1/mM^{m+}[A-CFX-SO_3]^-$, $1/mM^{m+}[A-CFX-SO_2-N-SO_2-R_F]^-$, $1/mM^{m+}[A-CFX-SO_2-C(SO_2-R_F)_2]^-$, $1/mM^{m+}[A-CFX-SO_2-CH(SO_2-R_F)]^-$ $1/mM^{m+}[A^1-CFX^1-SO_2-N-SO_2-CFX^2-A^2]^-$, $1/mM^{m+}[A^1-CFX^1-SO_2-CH(SO_2-CFX^2-A^2)]^-$, $1/mM^{m+}[A^1-CFX^1-SO_2-C(SO_2-CFX^2-A^2)_2]^-$,

dans lesquelles $A^1$ et $A^2$, identiques ou différents, ont la même signification que A ; $X^1$ et $X^2$, identiques ou différents, ont la même signification que X.

6. Procédé de préparation d'un composé selon la revendication 1 dans lequel Z représente [$-O^-$, $1/mM^{m+}$], caractérisé en ce qu'il consiste à faire réagir un anhydride cyclique

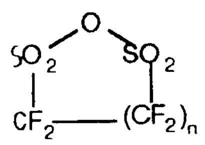

avec une amine $R^1R^2NH$, en présence d'une base, et à remplacer le groupement ($H^+$Base) du produit obtenu par $1/mM^{m+}$ par échange d'ions.

7. Procédé de préparation d'un composé selon la revendication 1, dans lequel Z représente un halogène, caractérisé en ce qu'il consiste à traiter un composé selon la revendication 1 dans lequel Z représente [$-O^-$,$1/mM^{m+}$] ou [$-O^-$,($H^+$base)] avec un agent d'halogénation.

8. Procédé de préparation d'un composé selon la revendication 1 dans lequel Z représente $-OSi(CH_3)_3$, caractérisé en ce qu'il consiste à faire réagir le composé $A-CFX-SO_2F$ avec un agent de silylation tel que l'hexaméthyldisiloxane.

9. Procédé de préparation d'un composé selon la revendication 1 dans lequel Z représente [$-N(SO_2-Q)^-$, $1/mM^{m+}$], [$-CH(SO_2-Q)^-$, $1/mM^{m+}$)] et [$-C(SO_2-Q)_2^-$, $1/mM^{m+}$], Q représentant $-R_F$ ou -CFX-A, caractérisé en ce qu'il consiste à faire réagir le composé $A-CFX-SO_2F$ avec respectivement $1/mM^{m+}[HNSO_2Q]^-$, $1/mM^{m+}[CH_2(SO_2Q)]^-$ ou $1/mM^{m+}[HC(SO_2Q)_2]^-$, en présence d'une base nucléophile.

10. Procédé de préparation d'un composé selon la revendication 1 présentant une structure symétrique telle que $1/mM^{m+}[(A-CFX-SO_2)_2N]^-$ ou $1/mM^{m+}[(A-CFX-SO_2)_2CH]^-$ ou $1/mM^{m+}$ $[(A-CFX-SO_2)_3C]^-$, caractérisé en ce qu'il consiste à faire réagir le composé $A-CFX-SO_2F$ respectivement avec le nitrure ionique $Li_3N$ ou le carbure ionique $C_3Al_4$ dans un solvant aprotique polaire.

11. Polymère obtenu par polymérisation d'un composé selon la revendication 1.

12. Polymère selon la revendication 11, caractérisé en ce que Z représente un groupement ionique choisi parmi [$-N(SO_2-Q)^-$, $1/mM^{m+}$], [$-CH(SO_2-Q)^-$, $1/mM^{m+}$)] et [$-C(SO_2-Q)_2^-$ , $1/mM^{m+}$], Q représentant $-R_F$ ou -CFX-A ; $M^{m+}$ représentant un ion d'un métal ayant la valence m, choisi parmi les métaux alcalins, les métaux alcalino-terreux, les métaux de transition et les terres rares, ou un ion hydronium, ou un ion ammonium répondant à la formule $NH_{(4-j)}R_j^+$, ou un ion amidinium répondant à la formule $RC(NH_{2-j}R_j)_2^+$, ou un ion guanidinium répondant à la formule $C(NH_{2-j}R_j)_3^+$, avec j = 0, 1 ou 2, R étant choisi parmi l'hydrogène, un groupement alkyle, oxaalkyle ou aryle.

13. Polymère selon la revendication 11, caractérisé en ce qu'il est obtenu par copolymérisation avec au moins un autre monomère.

14. Réseau macromoléculaire obtenu par coréticulation d'un monomère selon la revendication 1 avec un polymère préexistant.

15. Matériau à conduction ionique contenant un polymère selon l'une des revendications 12 ou 13, ou un réseau macromoléculaire selon la revendication 14.

16. Application d'un matériau selon la revendication 15 à la réalisation d'un dispositif électrochimique.

## Claims

1. A compound having the general formula (1)

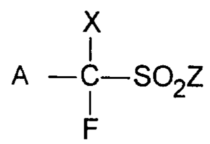

wherein :

- A represents

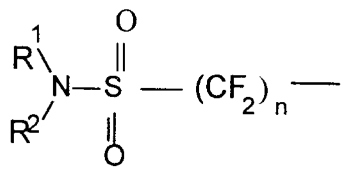

- Z denotes F, Cl, $-OSi(CH_3)_3$ or an ionic group,
- X denotes F, Cl, H or $R_F$ ;
- at least one of the groups $R^1$ and $R^2$ is a polymerisable non perflurorinated organic radical, wherein $R^1$ or $R^2$ are nonperfluoninated organic radicals when non polymerizable ;
- $R_F$ is a perfluoroalkyl radical or a perfluoroaryl radical;
- n = 1, 2 or 3, the preferred values being 1 or 2 when X = F.

**2.** A compound according to claim 1, characterised in that Z represents an ionic group selected from among $[-O^-, 1/mM^{m+}]$, $[-N(SO_2-Q)^-, 1/mM^{m+}]$, $[-CH(SO_2-Q)^-, 1/mM^{m+}]^-$ and $[-C(SO_2-Q)_2^-, 1/mM^{m+}]^-$, Q represents $-R_F$ or -CFX-A ; $M^{m+}$ represents an ion of a metal having the valency m and chosen from among alkali metals, alkaline earth metals, transition metals and rare earths, or hydronium ion or an ammonium ion having the formula $NH_{(4-j)}R_j^+$, or an amidinium ion having the formula $RC(NH_{2-j}R_j)_2^+$, or a guanidinium ion having the formula $C(NH_{2-j}R_j)_3^+$, wherein j = 0, 1 or 2, R is chosen from among hydrogen, an alkyl group, an oxaalkyl group or an aryl group.

**3.** A compound according to claim 1 or 2, characterised in that $R_F$ is selected from among perfluoroalkyl groups having 1 to 8 carbone atoms, and perfluoroaryl groups having 6 to 8 carbone atoms.

**4.** A compound according to claim 1, characterised in that $R^1$ or $R^2$ comprise double bonds such as vinyl, allyl, vinylbenzyl or acryloyl, or an oxirane group, an oxetane group, an azetidine group, an aziridine group, an alcohol group, a thiol group, an amine group, an isocyanate group or a trialcoxysilane group.

**5.** A compound according to claim 1, characterised in that it has one of the following formulaes :

$1/mM^{m+}[A-CFX-SO_3]^-$, $1/mM^{m+}[A-CFX-SO_2-N-SO_2-R_F]^-$, $1/mM^{m+}[A-CFX-SO_2-C(SO_2-R_F)_2]^-$, $1/mM^{m+}[A-CFX-SO_2-CH(SO_2-R_F]^-$ $1/mM^{m+}[A^1-CFX^1-SO_2-N-SO_2-CFX^2-A^2]^-$, $1/mM^{m+}[A^1-CFX^1-SO_2-CH(SO_2-CFX^2-A^2)]^-$, $1/mM^{m+}[A^1-CFX^1-SO_2-C(SO_2-CFX^2-A^2)_2]^-$,

wherein $A^1$ and $A^2$, which are identical or different, have the meaning previously given for A ; $X^1$ et $X^2$, which are identical or different, have the meaning previously given for X.

**6.** A process for the preparation of a compound according to claim 1 wherein Z represents $[-O^-, 1/mM^{m+}]$, characterised in that it consists in reacting a cyclic anhydride

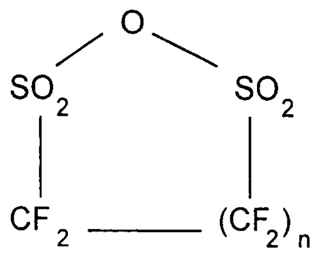

with an amine $R^1R^2NH$, in the presence of a base, and in replacing in the product which is obtained, ($H^+$Base) by $1/mM^{m+}$ by ion exchange.

7. A process for the preparation of a compound according to claim 1, where Z represents an halogenide, characterised in that it consists in reacting a compound according to claim 1 wherein Z represents [$-O^-$,$1/mM^{m+}$] or [$-O^-$, ($H^+$base)] with an halogenating agent.

8. A process for the preparation of a compound according to claim 1 wherein Z represents $-OSi(CH_3)_3$, characterised in that it consists in reacting the compound A-CFX-$SO_2F$ with a silylating agent such as hexamethyldisiloxane.

9. A process for the preparation of a compound according to claim 1 wherein Z represents [$-N(SO_2-Q)^-$, $1/mM^{m+}$], [$-CH(SO_2-Q)^-$, $1/mM^{m+}$] and [$-C(SO_2-Q)_2^-$, $1/mM^{m+}$], Q represents $-R_F$ or -CFX-A, characterised in that it consists in reacting the compound A-CFX-$SO_2F$ respectively with $1/mM^{m+}[HNSO_2Q]^-$, $1/mM^{m+}[CH_2(SO_2Q)]^-$ or $1/mM^{m+}[HC(SO_2Q)_2]^-$, in the presence of a nucleophilic base.

10. A process for the preparation of a compound according to claim 1 having a symmetrical structure such as $1/mM^{m+}[(A\text{-}CFX\text{-}SO_2)_2N]^-$ or $1/mM^{m+}[(A\text{-}CFX\text{-}SO_2)_2CH]^-$ or $1/mM^{m+}$ $[(A\text{-}CFX\text{-}SO_2)_3C]^-$, characterised in that it consists in reacting the compound A-CFX-$SO_2F$ respectively with the ionic nitride $Li_3N$ or the ionic carbide $C_3Al_4$ in a aprotic polar solvent.

11. A polymer obtained by polymerisation of a compound according to 1.

12. A polymer according to claim 11, characterised in that Z represents an ionic group selected from among [$-N(SO_2-Q)^-$, $1/mM^{m+}$], [$-CH(SO_2-Q)^-$, $1/mM^{m+}$] and [$-C(SO_2-Q)_2^-$, $1/mM^{m+}$], wherein Q represents $-R_F$ or -CFX-A ; $M^{m+}$ represents an ion of a metal having the valency m and chosen from among alkali metals, alkaline earth metals, transition metals and rare earths, or a hydronium ion, or an ammonium ion having the formula $NH_{(4-j)}R_j^+$, or an amidinium ion having the formula $RC(NH_{2-j}R_j)_2^+$, or a guanidinium ion having the formula $C(NH_{2-j}R_j)_3^+$, wherein j = 0, 1 or 2, R is chosen from among hydrogen, an alkyl group, an oxaalkyl group or an aryl group.

13. A polymer according to claim 11, characterised in that it is obtained by copolymerisation with at least one other monomer.

14. A macromolecular network obtaind by co-crosslinking a monomer according to claim 1 with a pre-existing polymer.

15. An ion-conducting material containing a polymer according to claim 12 or 13, or a macromolecular network according to claim 14.

16. Use of a material according to claim 15 for making an electrochemical device.

**Patentansprüche**

1. Verbindung der allgemeinen Formel

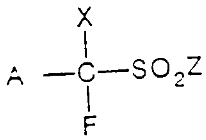

worin:

- A für

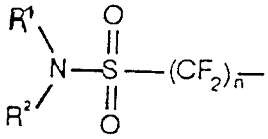

steht;

- Z für F, Cl, $-OSi(CH_3)_3$ oder eine Ionengruppe steht;
- X für F, Cl, H oder $R_F$ steht;
- zumindest einer der Reste $R^1$ und $R^2$ aus den organischen, nicht-perfluorierten, polymerisierbaren Resten ausgewählt ist, wobei $R^1$ oder $R^2$ ein organischer, nichtperfluorierter Rest ist, wenn er nicht-polymerisierbar ist; $R_F$ aus den Perfluoralkyl-Resten und Perfluoraryl-Resten ausgewählt ist; n = 1, 2 oder 3, wobei die bevorzugten Werte 1 oder 2 sind, wenn X = F.

**2.** Verbindung nach Anspruch 1, dadurch gekennzeichnet, daß Z eine Ionengruppe darstellt, die aus $[-O^-, 1/mM^{m+}]$, $[-N(SO_2-Q)^-, 1/mM^{m+}]$, $[-CH(SO_2-Q)^-, 1/mM^{m+}]^-$ und $[-C(SO_2-Q)_2^-, 1/mM^{m+}]^-$ ausgewählt ist, Q $-R_F$ oder -CFX-A darstellt; $M^{m+}$ ein Ion eines Metalls mit der Wertigkeit m, ausgewählt aus Alkalimetallen, Erdalkalimetallen, Übergangsmetallen und Seltenerden, oder ein Hydronium-Ion, oder ein Ammonium-Ion, das der Formel $NH_{(4-j)}R_j^+$ entspricht, oder ein Amidinium-Ion, das der Formel $RC(NH_{2-j}R_j)_2^+$ entspricht, oder ein Guanidinium-Ion, das der Formel $C(NH_{2-j}R_j)_3^+$ entspricht, darstellt, wobei j = 0, 1 oder 2 und R aus Wasserstoff, einer Alkyl-, Oxalkyl- oder Arylgruppe ausgewählt ist.

**3.** Verbindung nach einem der Ansprüche 1 oder 2, dadurch gekennzeichnet, daß $R_F$ aus den Perfluoralkyl-Gruppen mit 1 bis 8 Kohlenstoffatomen und den Perfluoraryl-Gruppen mit 6 bis 8 Kohlenstoffatomen ausgewählt ist.

**4.** Verbindung nach Anspruch 1, dadurch gekennzeichnet, daß die Gruppen $R^1$ oder $R^2$ Doppelbindungen des Vinyl-, Allyl-, Vinylbenzyl- oder Acryloyl-Typs oder eine Oxiran-, Oxetan-, Azetidin-, Aziridin-, Alkohol-, Thiol-, Amin-, Isocyanat- oder Trialkoxysilan-Funktion tragen.

**5.** Verbindung nach Anspruch 1, dadurch gekennzeichnet, daß sie einer der folgenden Formeln entspricht:

$1/mM^{m+}[A-CFX-SO_3]^-$, $1/mM^{m+}[A-CFX-SO_2-N-SO_2-R_F]^-$, $1/mM^{m+}[A-CFX-SO_2-C(SO_2-R_F)_2]^-$, $1/mM^{m+}[A-CFX-SO_2-CH(SO_2-R_F)]^-$ $1/mM^{m+}[A^1-CFX^1-SO_2-N-SO_2-CFX^2-A^2]^-$, $1/mM^{m+}[A^1-CFX^1-SO_2-CH(SO_2-CFX^2-A^2)]^-$, $1/mM^{m+}[A^1-CFX^1-SO_2-C(SO_2-CFX^2-A^2)_2]^-$,

worin $A^1$ und $A^2$, die gleich oder unterschiedlich sind, die gleiche Bedeutung haben wie A und $X^1$ und $X^2$, die gleich oder unterschiedlich sind, die gleiche Bedeutung haben wie X.

**6.** Verfahren zur Herstellung einer Verbindung nach Anspruch 1, worin Z $[-O^-, 1/mM^{m+}]$ darstellt, dadurch gekennzeichnet, daß es das Umsetzen eines zyklischen Anhydrids

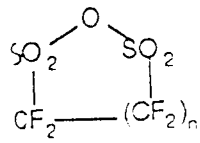

mit einem Amin $R^1R^2NH$ in der Gegenwart einer Base und das Ersetzen der Gruppe ($H^+$-Base) des durch $1/mM^{m+}$ erhaltenen Produkts durch Ionenaustausch umfaßt.

**7.** Verfahren zur Herstellung einer Verbindung nach Anspruch 1, worin Z ein Halogen darstellt, dadurch gekennzeichnet, daß es das Behandeln einer Verbindung nach Anspruch 1, in der Z [$-O^-$, $1/mM^{m+}$] oder [-O, ($H^+$-Base)] darstellt, mit einem Halogenierungsmittel umfaßt.

**8.** Verfahren zur Herstellung einer Verbindung nach Anspruch 1, worin Z $-OSi(CH_3)_3$ darstellt, dadurch gekennzeichnet, daß es das Umsetzen der Verbindung A-CFX-$SO_2F$ mit einem Silylierungsmittel wie z.B. Hexamethyldisiloxan umfaßt.

**9.** Verfahren zur Herstellung einer Verbindung nach Anspruch 1, worin Z [$-N(SO_2-Q)^-$, $1/mM^{m+}$], [$-CH(SO_2-Q)^-$, $1/mM^{m+}$)] und [$-C(SO_2-Q)_2^-$, $1/mM^{m+}$] darstellt, sowie Q $-R_F$ oder -CFX-A darstellt, dadurch gekennzeichnet, daß es das Umsetzen der Verbindung A-CFX-$SO_2F$ mit $1/mM^{m+}[HNSO_2Q]^-$, $1/mM^{m+}[CH_2(SO_2Q)]^-$ bzw. $1/mM^{m+}[HC(SO_2Q)_2]^-$in Gegenwart einer nukleophilen Base umfaßt.

**10.** Verfahren zur Herstellung einer Verbindung nach Anspruch 1, die eine symmetrische Struktur wie z.B. $1/mM^{m+}[A\text{-}CFX\text{-}SO_2)_2N]^-$ oder $1/mM^{m+}[(A\text{-}CFX\text{-}SO_2)_2CH]^-$ oder $1/mM^{m+}[(A\text{-}CFX\text{-}SO_2)_3C]^-$ aufweist, dadurch gekennzeichnet, daß es das Umsetzen der Verbindung A-CFX-$SO_2F$ mit dem ionsichen Nitrid $Li_3N$ oder dem ionischen Carbid $C_3Al_4$ in einem aprotischen polaren Lösungsmittel umfaßt.

**11.** Polymer, das durch Polymerisation einer Verbindung nach Anspruch 1 erhalten worden ist.

**12.** Polymer nach Anspruch 11, dadurch gekennzeichnet, daß Z eine Ionengruppe darstellt, die aus [$-N(SO_2-Q)^-$, $1/mM^{m+}$], [$-CH(SO_2-Q)^-$, $1/mM^{m+}$)] und [$-C(SO_2-Q)_2^-$, $1/mM^{m+}$] ausgewählt ist, worin Q $-R_F$ oder -CFX-A darstellt; $M^{m+}$ ein Ion eines Metalls mit der Wertigkeit m darstellt, ausgewählt aus Alkalimetallen, Erdalkalimetallen, Übergangsmetallen und Seltenerden, oder ein Hydronium-Ion, oder ein Ammonium-Ion, das der Formel $NH_{(4-j)}R_j^+$ entspricht, oder ein Amidinium-Ion, das der Formel $RC(NH_{2-j}R_j)_2^+$ entspricht, oder ein Guanidinium-Ion, das der Formel $C(NH_{2-j}R_j)_3^+$ entspricht, darstellt, wobei j = 0, 1 oder 2 darstellt und R aus Wasserstoff, einer Alkyl-, Oxalkyl- oder Arylgruppe ausgewählt ist.

**13.** Polymer nach Ansrpuch 11, dadurch gekennzeichnet, daß es durch Copolymerisation mit zumindest einem weiteren Monomer erhalten worden ist.

**14.** Makromolekulares Netzwerk, das durch Covernetzung eines Monomers nach Anspruch 1 mit einem bereits bestehenden Polymer erhalten worden ist.

**15.** Ionisch leitendes Material, das ein Polymer nach einem der Ansprüche 12 oder 13 enthält, oder ein makromolekulares Netzwerk nach Anspruch 14.

**16.** Verwendung eines Materials nach Anspruch 15 zur Gestaltung einer elektrochemischen Vorrichtung.